# EUROPÄISCHE PATENTSCHRIFT

(11) **EP 2 095 834 B1**
(45) Veröffentlichungstag und Bekanntmachung des Hinweises auf die Patenterteilung: **01.07.2015**
(21) Anmeldenummer: 09002310.2
(22) Anmeldetag: 19.02.2009
(51) Int. Cl.: A61L 31/12, A61L 31/16

(54) **Prophylaxeartikel**
Prophylaxis items
Article prophylactique

(30) Priorität: 21.02.2008 AT 2792008; 21.02.2008 US 30350 P
(43) Veröffentlichungstag der Anmeldung: 02.09.2009
(73) Patentinhaber: Semperit Aktiengesellschaft Holding, 1031 Wien (AT)
(72) Erfinder: Schaller, Raimund, Dipl.-Ing.. Dr., 2620 Neunkirchen (AT)
(74) Vertreter: Ofner, Clemens

(56) Entgegenhaltungen:
- AT-B- 413 471
- DE-A1-102005 059 721
- US-A- 5 138 719
- US-A1- 2005 066 414

## Beschreibung

Die Erfindung betrifft einen Prophylaxeartikel, insbesondere einen Handschuh, mit einem Trägerelement, das zumindest teilweise aus einem natürlichen oder synthetischen Elastomer besteht, sowie mit einer Wirkstoffkombination, die in Mikrokapseln enthalten ist, wobei die Mikrokapseln zumindest teilweise im und/oder auf dem Trägerelement, insbesondere im Bereich der inneren, dem Träger zugewandten Oberfläche, angeordnet sind.

Medizinische Handschuhe, sei es in Form von Untersuchungshandschuhen oder in Form von Operationshandschuhen, gehören mittlerweile zum standardmäßigen Equipment der medizinischen Versorgung. Problematisch ist hierbei, dass die Haut des Verwenders unter Umständen mit Irritationen oder allergisch auf die diversen Elastomermaterialien reagiert, wenn derartige Handschuhe über einen längeren Zeitraum getragen werden. Um dies zu vermeiden, wurde im Stande der Technik bereits vorgeschlagen, diverse Wirkstoffe über den Handschuh auf die Haut aufzubringen.

So ist z.B. aus der US 6,274,154 B1 ein therapeutischer Handschuh bekannt, der an seiner inneren Oberfläche eine Schicht aus dehydratisierter Aloe Vera aufweist.

Ähnlich dazu beschreibt die US 4,775,372 A einen Aloe Vera Öl enthaltenden Handschuh, wobei das Öl zwischen zwei Schichten aus flexiblen Kunststoff angeordnet ist. Nachteilig hierbei ist jedoch, dass zur Applikation des Öls eine der Schichten punktiert werden muss, wodurch der Handschuh nicht nur seine Festigkeit zumindest bereichsweise verliert, sondern damit auch die Gefahr verbunden ist, dass Substanzen von außen in die Haut des Verwenders eindringen.

Im Stand der Technik wurde bereits auch die Mikroverkapselung von Wirkstoffen beschrieben, bspw. in der DE 201 00 269 U1 der in der auf die Anmelderin zurückgehenden AT 413 471 B bzw. AT 503 090 A.

Da das Fassungsvermögen von Mikrokapseln naturgemäß sehr gering ist, ist es von Bedeutung, dass die darin enthaltenen Wirkstoffe möglichst rasch und vollständig von der Haut des Trägers der Handschuhe aufgenommen werden.

Die DE 10 2005 059 721 A1 beschreibt ein Verfahren zur Ausrüstung von Textilien mit pflegenden Ölen, wobei man wässrige Emulsionen mit einer Viskosität von unterhalb 200 mPas (gemessen nach Brookfield bei 20 °C) enthaltend Wasser, ein oder mehrere pflegende Öle und ein oder mehrere Emulgatoren durch Versprühen auf das Textil aufbringt. Die Emulsionen kann zusätzlich Mikrokapseln enthalten, in denen Duftstoffe, wie Parfümöle, oder pflegend wirkende Substanzen enthalten sind.

Es ist daher Aufgabe der Erfindung, einen Handschuh zur Verfügung zu stellen, der verbesserte Hautpflegeeigenschaften aufweist.

Diese Aufgabe der Erfindung wird durch den eingangs genannten Prophylaxeartikel gelöst, bei dem die Wirkstoffkombination zumindest ein Hautpflegemittel, zumindest ein Konservierungsmittel, zumindest ein geruchshemmendes Mittel und zumindest ein Antioxidans enthält, wobei das zumindest eine Hautpflegemittel ausgewählt ist aus einer Gruppe umfassend Triglyceride von Kokosfettsäuren, Avocadoöl, Jojobaöl, Olivenöl, Cyclomethicone, Squalan, Borretschöl, Sheabutter, Macadamiaöl, das zumindest eine Konservierungsmittel ausgewählt ist aus einem Gruppe umfassend Benzylalkohohl, Benzylbenzoat, Kaliumsorbat, Natriumhydroacetat, das zumindest eine geruchshemmende Mittel ausgewählt ist aus einer Gruppe umfassend Citral, Cumarin, Citronellol, Lilial, Linalool, Limonene, das zumindest eine Antioxidans ausgewählt ist aus einer Gruppe umfassend Tocopheryl Acetat, Ascorbylpalmitat, Betacaroten, Rosmarinöl, und wobei die Gruppen auch zumindest partiell hydratisierte Vertreter oder Extrakte der genannten Wirkstoff umfassen und wobei die Wirkstoffkombination eine Verseifungszahl nach DIN 53401 von max. 250 aufweist.

Im Zuge der Weiterentwicklung des Produktes der Anmelderin entsprechend den beiden oben stehend zitierten Dokumenten zum Stand der Technik wurde überraschenderweise festgestellt, dass die neue Wirkstoffkombination nicht nur besonders hautverträglich ist, sondern diese rasch in die Haut des Benutzers des Prophylaxeartikels eindringt. In Kombination mit der Mikroverkapselung wird ein Prophylaxeartikel erhalten, der seine Wirkstoffe, d.h. die hautpflegenden Substanzen, über einen sehr langen Zeitraum dem Benutzer zuführt, da die Mikrokapseln nicht alle zur selben Zeit aufbrechen, insbesondere beim Anziehen der Handschuhe, sondern die Wirkstoffe erst nach und nach aufgrund der ständigen mechanischen Beanspruchung der Handschuhe freigesetzt werden. Damit wird die Pflegewirkung über einen längeren Zeitraum aufrechterhalten, wobei durch das rasche Einziehen der Wirkstoffe in die Haut kaum bzw. keine Verluste an Wirkstoffen auftreten. Es ist damit möglich, den Anteil der Wirkstoffe am gesamten Handschuh, im Vergleich zu Komplettbeschichtungen von Handschuhen, zu reduzieren, wodurch die Handschuhe im Vergleich zu Stand der Technik Produkten mit Beschichtungen kostengünstiger herstellbar sind. Es hat sich weiters herausgestellt, dass diese Substanzen besonders gut mikroverkapselt werden können, wodurch das Herstellungsverfahren an sich kostengünstiger gestaltet werden kann, womit wiederum positive Auswirkungen auf die Gestehungskosten des Prophylaxeartikels resultieren. Durch die Beimengung eines Konservierungsmittels sowie zumindest eines Antioxidans wird dieser Wirkstoffkombination eine höhere Haltbarkeit verliehen, sodass die Prophylaxeartikel auch nach längerer Lagerzeit noch ihre volle Wirkung entfalten. Mit Hilfe des geruchshemmenden Mittels, welches maskierend auf Geruchsmoleküle wirkt, wird einerseits erreicht, dass der Grundgeruch des Produktes selbst zumindest annähernd neutralisiert werden kann, andererseits entfaltet dieses Mittel auch seine Wirkung während des Tragens der Prophylaxeartikel. Es kann damit Geruchsbildungen aufgrund Schwitzen des Trägers - da es sich um einen Prophylaxeartikel handelt muss dieser undurchlässig für Flüssigkeiten und Gase sein - vorgebeugt werden kann. Durch die Applikation des Hautpflegemittels wird die Haut des Trägers des Prophylaxeartikels geschmeidiger und wird diese geglättet, was insbesondere in Hinblick auf die Ausbildung des Prophylaxeartikels als medizinischer Handschuh von Bedeutung ist, da durch das oftmalige Waschen der Hände, zu welchem Ärzte, insbesondere Chirurgen, angehalten sind, diese häufig dazu neigt, auszutrocknen.

Um ein hohes Lipidpenetrationsvermögen der Wirkstoffkombination bzw. einzelner Bestandteile hiervon zu erreichen, ist es von Vorteil, wenn diese Wirkstoffkombination eine Viskosität bei 37°C von max. 250 mPas, insbesondere max. 100 mPas, aufweist und die Wirkstoffkombination eine Verseifungszahl nach DIN 53401 von max. 250, insbesondere max. 150, aufweist.

Wie bekannt ist die Verseifungszahl ein Maß für die in 1g Fett vorkommenden, gebundenen und freien Säuren. Sie gibt an, wie viel mg einer Lauge notwendig sind, um die in 1g Fett enthaltenen freien Säuren zu neutralisieren und die vorhandenen Esterbindungen zu spalten.

Nach einer Ausführungsvariante der Erfindung ist vorgesehen, dass das oder die Konservierungsmittel, das oder die geruchshemmende(n) Mittel und das Antioxidans oder die Antioxidantien in Summe in einem Anteil von max. 10 Gew.-% enthalten sind und den Rest das oder die Hautpflegemittel bilden. Besonders bevorzugt ist dabei, wenn dieser Summenanteil max. 5 Gew.-% beträgt. Diese Reduzierung dieser Inhaltsstoffe der Wirkstoffkombination ist möglich, da die Wirkstoffkombination mikroverkapselt ist, d.h. die Wirkstoffe an sich durch die Kapselhülle bereits geschützt ist. Es ist somit möglich, den hautpflegenden Anteil an der Wirkstoffkombination entsprechend zu erhöhen, wodurch nicht nur die hautpflegende Wirkung der damit ausgerüsteten Prophylaxeartikel erhöht werden kann, sondern wird durch die Reduzierung der "aggressiver" wirkenden Substanzen - im Vergleich zu Hautpflegemittel - einer ggf. auftretenden Hautunverträglichkeit besser vorgebeugt.

Es kann weiters vorgesehen sein, dass die Wirkstoffkombination zumindest einen Feuchthaltefaktor enthält, der ausgewählt ist aus einer Gruppe umfassend Bisabolol, Fructose, Inositol, Nicotinsäureamid, Natriumlaktat. Durch diese Weiterbildung des Prophylaxeartikels wird eine bessere Verträglichkeit für Benutzer mit relativ trockener Haut erreicht. Wiederum ist bei den ausgewählten Feuchthaltefaktoren von Vorteil, dass diese sehr rasch ihre Wirkung entfalten und zudem keine störenden Interaktionen mit den Hautpflegemitteln auftreten, wodurch die Verarbeitbarkeit, insbesondere die Mikroverkapselung, verbessert ist. Zudem kann damit ein möglicherweise auftretendes Unbehagen an der Haut, die Prophylaxeartikel liegen normalerweise sehr eng an der Haut an - insbesondere bei Handschuhen werden häufig Größen verwendet, die eine Nummer kleiner ist, als dies der Handgröße entsprechen würde, um die Taktilität zu verbessern - gelindert werden.

Durch die rasche Verfügbarkeit des Feuchthaltefaktors kann dessen Anteil an der Wirkstoffkombination wiederum sehr gering gehalten werden, sodass dieser Anteil des Feuchthaltefaktors oder der Feuchthaltefaktoren in Summe max. 3 Gew.-%, vorzugsweise max. 1,5 Gew.-% betragen kann.

Es sei an dieser Stelle erwähnt, dass sämtliche weiteren Wirkstoffe die neben dem Hautpflegemittel, dem geruchshemmenden Mittel, dem Antioxidans bzw. dem Konservierungsmittel im Prophylaxeartikel enthalten sind, eine Reduktion des Anteils des Hautpflegemittels oder eine Reduktion des Anteils an Konservierungsmittel, Antioxidans und geruchshemmenden Mittel bedingt.

Weiters kann die Wirkstoffkombination Geraniol enthalten, wodurch ein angenehmes Gefühl an der Haut während des Tragens des Prophylaxeartikels erzeugt werden kann, damit also die Tragbarkeit des Prophylaxeartikels über einen längeren Zeitraum verbessert wird.

Der Anteil an Geraniol in der Wirkstoffkombination kann dabei vorzugsweise max. 0,3 Gew.-% insbesondere max. 0,1 Gew.-% betragen, da die erfindungsgemäße Wirkstoffkombination an sich bereits eine sehr gute Hautverträglichkeit aufweist und damit ein möglicherweise auftretendes irritierendes Gefiihl auf der Haut bereits reduziert ist.

Die Wirkstoffkombination kann zumindest einen weiteren Wirkstoff ausgewählt aus einer Gruppe umfassend Allantonin, Arnikaextrakt, Haferextrakt, Ringelblumenöl, Kamillenextrakt, Ethylhexylstearat, Azulen, enthalten. Es können damit dem Prophylaxeartikel im gewissen Umfang hautregenerierende, durchblutungsfördernde, juckreizstillende Wirkung verliehen werden. Insbesondere durch Ethylhexylstearat wird die Verteilbarkeit der Wirkstoffkombination auf der Haut verbessert.

Der Anteil des zumindest einen weiteren Wirkstoffes an der Wirkstoffkombination kann in Summe bevorzugt max. 10 Gew.-% betragen, insbesondere max. 5 Gew.-%, vorzugsweise max. 2,5 Gew.-%.

Es ist weiters möglich, der Wirkstoffkombination zumindest ein Parfum zuzusetzen, um deren Eigengeruch zu überdecken bzw. um den Prophylaxeartikel in unterschiedlichsten Geruchsvariationen zur Verfügung zu stellen.

Zur Verbesserung der Verkapselbarkeit dieser Wirkstoffkombination ist gemäß einer Ausführungsvariante vorgesehen, dass diese mit einem Triglycerid und einem Duftstoff verdünnt wird, wobei der Anteil des Triglycerids ausgewählt ist aus einem Bereich mit einer unteren Grenze von 10 Gew.-% und einer oberen Grenze von 20 Gew.-%, vorzugsweise mit einer unteren Grenze von 12 Gew.-% und einer oberen Grenze von 16 Gew.-%, der Anteil des Duftstoffes gewählt ist aus einem Bereich mit einer unteren Grenze von 5 Gew.-% und einer oberen Grenze von 15 Gew.-%, vorzugsweise aus einem Bereich mit einer unteren Grenze von 8 Gew.-% und der oberen Grenze von 12 Gew.-%, und dem Rest die Wirkstoffkombination bildet.

Dieser Duftstoff kann dabei durch Lavendelöl gebildet sein, wodurch nicht nur der Ölcharakter der Wirkstoffkombination erhöht wird, sondern dem Prophylaxeartikel aufgrund der Wirkung des Lavendelöls auch eine antiseptische und beruhigende Wirkung verliehen wird.

Zur Verbesserung der Anziehbarkeit des Prophylaxeartikels, insbesondere in der Ausbildung als medizinischer Handschuh, kann das Trägerelement auf einer Oberfläche, d. h. der inneren Oberfläche des Handschuhs, eine Gleitschicht aufweisen, da wie bereits erwähnt, medizinische Handschuhe sehr eng anliegend ausgeführt sind und damit insbesondere beim Anziehen mit nassen Händen Probleme hinsichtlich der Gleitfähigkeit des Elastomers auf der Haut auftreten können.

Es ist dabei von Vorteil, wenn die Mikrokapseln zumindest teilweise in und/oder auf der Gleitschicht angeordnet sind, wodurch die Applikation der Wirkstoffkombination auch bei dieser Ausführungsvariante verbessert ist. Auch in diesem Fall können zusätzlich unterhalb der Gleitschicht, d.h. zumindest teilweise in und/oder auf dem Elastomer des Trägerelementes, Mikrokapseln angeordnet sein, bzw. besteht die Möglichkeit, dass die Mikrokapseln ausschließlich in und/oder auf der Gleitschicht angeordnet sind.

Bevorzugt weist die Wirkstoffkombination eine zumindest annähernd ölartige Konsistenz auf, insbesondere ist diese Wirkstoffkombination ein Hautschutzöl, wodurch die Applikation der Wirkstoffe in tiefere Regionen der Haut rascher erfolgt und damit wiederum eine Reduzierung des Anteils der Wirkstoffkombination am Prophylaxeartikel erreicht werden kann.

Um ein hohes Lipidpenetrationsvermögen der Wirkstoffkombination bzw. einzelner Bestandteile hiervon zu erreichen, ist es von Vorteil, der Anteil triglyceridischer Hautpflegemittel an der Wirkstoffkombination max. 50 Gew.-%, vorzugsweise max. 30 Gew.-%, beträgt. Durch die Reduktion des Anteils triglyceridischer Hautpflegemittel wird zum Ausdruck gebracht, dass die molekulare Konfiguration der Wirkstoffe vorzugsweise gerade Ketten und verzweigte Ester aufweist, die besser penetrieren als Triglycerid-Öle. Wiederum wird durch diese Maßnahmen eine Reduktion des Anteils der Wirkstoffkombination am Prophylaxeartikel erreicht, wodurch neben der Reduktion der Kosten auch eine höhere Sicherheit der Wirkstoffapplikation erhalten werden kann, für den Fall das einzelne Mikrokapseln nicht bersten und damit die Wirkstoffe nicht freisetzen. Zudem kann damit der Anteil der Mikrokapseln verringert werden, wodurch deren Homogenisierung im Elastomer während der Herstellung des Prophylaxeartikels erleichtert werden kann.

Zum besseren Verständnis der Erfindung wird diese anhand der nachfolgenden Beschreibung näher erläutert.

Bei dem erfindungsgemäßen Prophylaxeartikel kann es sich neben Handschuhen, insbesondere medizinischen Operations- oder Untersuchungshandschuhen, auch um Kondome, Fingerlinge oder Schutzhandschuhe für die Arbeit in Reinraumbereichen, etc. handeln. Derartige Prophylaxeartikel wurden bereits in der AT 413 471 B bzw. der AT 503 090 A der Anmelderin hinsichtlich ihrer Zusammensetzung betreffend das Elastomer, d.h. der natürlichen bzw. synthetischen Latices, bspw. Naturkautschuk, Polychloropren, synthetisches Polyisopren, Nitrilbutadein- und Styrolbutadienkautschuk, bzw. eine Mischung hiervon, sowie hinsichtlich der ggf. angeordneten Gleitschicht, wobei diese Gleitschicht durch einen Gleitfilm gebildet sein kann, bspw. ein Silikonöl, allerdings auch aus dem Latex selbst gebildet werden kann, bspw. durch Halogenierung, wie z.B. Chlorierung, oder durch bestimmte Oberflächenmodifikationen und -ausgestaltungen, ebenso wie hinsichtlich der verwendeten Mikrokapseln, insbesondere der Größe im Bereich zwischen 0,1 µm bis 100 µm, wobei im Rahmen der vorliegenden Erfindung vorzugsweise Mikrokapseln verwendet werden, die eine Größe ausgewählt aus einem Bereich zwischen 0,1 µm und 10 µm aufweisen, ebenso wie hinsichtlich des Kapselmaterials selbst, wobei hier vorzugsweise ein Polymer auf Melamin-Formaldehyd-Basis, bspw. entsprechend der DE 29 40 786 A, verwendet wird, ausreichend beschrieben. Auch die Applikation der Mikrokapseln selbst auf oder in dem Prophylaxeartikel durch Herstellung entsprechender Suspensionen bzw. Emulsionen aus den Latices, wurde in diesen Dokumenten bereits ausführlich abgehandelt, sodass eine eingehendere Erörterung an dieser Stelle nicht erforderlich ist, und wird daher auf diese beiden Dokumente zu diesen Punkten der Erfindung verwiesen, die somit diesbezüglich zum Inhalt der vorliegenden Erfindung gehören.

Vorzugsweise werden die Prophylaxeartikel im Tauchverfahren mit entsprechenden Tauchformen hergestellt. Diese können aber auch z.B. gesprüht werden, etc.

Die Mikroverkapselung der Wirkstoffkombination, insbesondere des Öls, hat den Vorteil, dass damit auch Wirkstoffe verwendet werden können, die hinsichtlich des Trägermaterials bzw. einer gegebenenfalls vorhandenen Gleitschicht unverträglich bzw. in geringerem Ausmaß verträglich sind. Mit anderen Worten weisen die Mikrokapseln eine Schutzfunktion für den bzw. die Werkstoff(e) des Prophylaxeartikels auf. Zudem kann damit verhindert werden, dass das Öl in den Prophylaxeartikel eindiffundiert und somit nur zu einem geringeren Ausmaß für die eigentliche Aufgabe zur Verfügung steht. Es wird also damit erreicht, dass die Wirkstoffkombination für die Haut des Trägers besser verfügbar ist.

Die nach der Erfindung vorgesehenen Inhaltsstoffe der Wirkstoffkombination wurden bereits voranstehend ausreichend erläutert. Es soll daher an dieser Stelle lediglich kurz die Einzelwirkung wiedergegeben werden, wobei angemerkt sei, dass es sich hierbei um übliche Wirkstoffe handelt, die in der Kosmetikindustrie verwendet werden und somit deren Wirkung an sich bereits ausreichend dokumentiert ist.

### Triglyceride von Kokosfettsäuren (Caprylic/Capric Triglyceride):

Dieser Wirkstoff macht die Haut geschmeidig und glättet sie. Er ermöglicht zudem eine gute Verteilbarkeit der Wirkstoffkombination auf der Haut. Der bevorzugte Anteil an der Wirkstoffkombination beträgt zwischen 25 Gew.-% und 50 Gew.-%, bzw. zwischen 30 Gew.-% und 40 Gew.-%.

### Avocadoöl (Persea Gratissima):

Dieser Wirkstoff macht die Haut geschmeidig und glättet sie. Zudem wird die Haut vor Austrocknung und spröde werden geschützt. Der bevorzugte Anteil beträgt zwischen 10 Gew.-% und 25 Gew.-% bzw. zwischen 15 Gew.-% und 20 Gew.-%.

### Jojobaöl (Simmondsia Chinensis, Buxus Chinensis):

Auch dieser Wirkstoff macht die Haut geschmeidig und glättet sie, ohne sichtbar zu fetten. Der bevorzugte Anteil beträgt 10 Gew.-% und 30 Gew.-% bzw. zwischen 15 Gew.-% und 20 Gew.-%.

### Olivenöl (Olea Europaea Fruitoil):

Olivenöl weist einen hohen Anteil an ungesättigten Fettsäuren zur Stärkung der Hautbarriere auf. Auch dieser Wirkstoff macht die Haut geschmeidig und glättet sie. Der bevorzugte Anteil beträgt zwischen 10 Gew.-% und 25 Gew.-% bzw. zwischen 15 Gew.-% und 20 Gew.-%.

### Cyclomethicone:

Wiederum hat dieser Wirkstoff- es handelt sich hierbei um eine Silikonölart - eine glättende Wirkung auf die Haut und macht diese geschmeidig. Zudem wirkt Cyclomethicone antistatisch, wodurch die Herstellung der Mikrokapseln verbessert werden kann, filmbildend, viskositätsregelnd und feuchtehaltend. Es handelt sich hierbei auch um einen Feuchthaltefaktor. Der bevorzugte Anteil beträgt zwischen 1 Gew.-% und 5 Gew.-%, bzw. 2 Gew.-% und 3 Gew.-%.

### Squalan:

Neben der Wirkung, dass dieser Wirkstoff die Haut geschmeidig macht und sie glättet, wirkt Squalan rückfettend, generell hautpflegend. Es handelt sich dabei um ein Extrakt aus Olivenöl. Der bevorzugte Anteil beträgt zwischen 1 Gew.-% und 5 Gew.-% bzw. zwischen 2 Gew.-% und 3 Gew.-%, kann in einzelnen Ausführungsbeispielen aber auch bis zu 20 Gew.-% betragen.

### Borretschöl (Borago Officinalis/Borage Se):

Borretschöl macht die Haut geschmeidig und glättet sie. Der bevorzugte Anteil beträgt zwischen 1 Gew.-% und 5 Gew.-% bzw. zwischen 2 Gew.-% und 3 Gew.-%, kann in einzelnen Ausführungsbeispielen aber auch bis zu 25 Gew.-% betragen.

### Sheabutter (Butyrospermum Parkii):

Sheabutter, bzw. ein flüssiges Extrakt hiervon, wirkt besonders pflegend und hautfreundlich. Der bevorzugte Anteil beträgt zwischen 1 Gew.-% und 5 Gew.-%, insbesondere zwischen 2 Gew.-% und 3 Gew.-%. Der Anteil kann aber auch bis zu 40 Gew.-% betragen.

### Macadamiaöl (Macadamia ternifolia):

Macadamiaöl hat eine hervorragende Pflegewirkung, da es reich an ungesättigten Fettsäuren ist. Der bevorzugte Anteil beträgt zwischen 1 Gew.-% und 5 Gew.-% bzw. zwischen 2 Gew.-% und 3 Gew.-%. Der Anteil kann aber auch bis zu 40 Gew.-% betragen.

### Benzylalkohol:

Benzylalkohol wirkt konservierend, hemmt in erster Linie die Entwicklung von Mikroorganismen in der Wirkstoffkombination. Darüber hinaus verleiht Benzylalkohol der Wirkstoffkombination auch einen jasminartigen Geruch. Der bevorzugte Anteil beträgt bis max. 0,5 Gew.-%, insbesondere bis max. 0,1 Gew.-%.

### Benzylbenzoat:

Benzylbenzoat ist ein sehr gut verträglicher Konservierungsstoff und hat ähnliche Wirkung wie Benzylalkohol allerdings ohne der Wirkstoffkombination einen bestimmten Geruch zu verleihen. Der bevorzugte Anteil beträgt bis max. 0,5 Gew.-% bzw. max. 0,1 Gew.-%.

### Kaliumsorbat:

Kaliumsorbat wird insbesondere verwendet für Prophylaxeartikel, die eine besonders hohe Hautverträglichkeit erfordern, d.h. insbesondere für Handschuhe, deren Träger sehr rasch mit einer Hautirritation auf Latices reagieren. Es handelt sich hierbei um einen milden Konservierungsstoff, der der Wirkstoffkombination eine längere Haltbarkeit verleiht. Der bevorzugte Anteil beträgt bis max. 0,5 Gew.-% bzw. max. 0,1 Gew.-%.

### Natriumhydroacetat:

Natriumhydroacetat ist ebenfalls ein gut verträglicher Konservierungsstoff ähnlich zu Kaliumsorbat. Der bevorzugte Anteil beträgt wiederum bis max. 0,5 Gew.-% bzw. max. 0,1 Gew.-%.

### Citral, Cumarin, Citronellol, Lilial (Butylphenyl Methylpropional):

Diese Wirkstoffe wirken maskierend, verhindern oder hemmen den Geruch der Wirkstoffkombination. Der bevorzugte Anteil beträgt bis max. 5 Gew.-% bzw. max. 0,5 Gew.-% bzw. max. 0,1 Gew.-% für jeden dieser Wirkstoffe, wenn sie einzeln in der Wirkstoffkombination enthalten sind.

### Linalool:

Linalool wird desodorierend. Der bevorzugte Anteil beträgt bis max. 0,5 Gew.-% bzw. bis max. 0,1 Gew.-%.

### Limonene:

Limonene wirkt hautpflegend und geruchshemmend. Der bevorzugte Anteil beträgt bis max. 0,5 Gew.-% bzw. bis max. 0,1 Gew.-%.

### Tocopheryl Acetat, Ascorbylpalmitat, Betacaroten, Rosmarinöl:

Es handelt sich hierbei um Antioxidantien, d. h. diese Wirkstoffe verhindern, dass einzelne Bestandteile der Wirkstoffkombination aufgrund längerer Lagerung oder aber auch bereits bei der Herstellung oxidieren und somit in ihrer Wirkung beeinträchtigt sind bzw. verhindern den Geruch von u.U. entstehenden, geruchsbildenden Abbauprodukten der Wirkstoffkombination. Es handelt sich hierbei zumeist um Radikalfänger. Der bevorzugte Anteil jedes dieser Vertreter der Antioxidanzien beträgt bis max. 0,5 Gew.-% bzw. bis max. 0,1 Gew.-%.

### Bisabolol, Fructose, Inositol, Nicotinisäureamid, Natriumlaktat:

Bei diesen Wirkstoffen handelt sich um Feuchthaltefaktoren, d.h. sie verhindern das Austrocknen der Haut. Jeder dieser Wirkstoffe kann bevorzugt in einem Anteil bis max. 3 Gew.-% bzw. bis max. 1 Gew.-% vorliegen, wobei diese Wirkstoffe in Summe in einem Anteil von max. 3 Gew.-% enthalten sind, wenn mehrere dieser Feuchthaltefaktoren verwendet werden. Die Feuchthaltefaktoren können insbesondere einzeln in einem Anteil 0,1 Gew.-% bis 1 Gew.-% enthalten sein.

### Geraniol:

Geraniol wirkt kräftigend und erzeugt ein angenehmes Gefühlt auf der Haut. Zudem kann es für eine Geruchskorrektur eingesetzt werden. Der bevorzugte Anteil beträgt bis max. 0,3 Gew.-% bzw. bis max. 0,1 Gew.-%.

### Allantonin, Arnikaextrakt (Arnica montana), Haferextrakt (Avena sativa), Ringelblumenöl (Calendula Officinalis), Kamillenextrakt (Chamomilla Recutita), Ethylhexylstearat, Azulen (Guajazulene):

Allantonin wirkt hautberuhigend und fördert die Bildung von Geweben, d.h. es hat eine positive Auswirkung, wenn der Träger der Prophylaxeartikel eine oberflächliche Hautverletzung hat.

Arnikaextrakt wird regenerierend und durchblutungsanregend.

Haferextrakt hat eine mild pflegende Wirkung, wirkt hautberuhigend und juckreizstillend.

Ringelblumenöl wird hautberuhigend und regenerierend bei aufgesprungener und rissiger Haut.

Kamillenextrakt wird insbesondere dann verwendet, wenn der Träger des Prophylaxeartikels eine sehr zarte, empfindliche Haut hat.

Ethylhexylstearat verbessert die Verteilbarkeit der Wirkstoffkombination auf der Haut.

Azulen ist ein hautberuhigender Wirkstoff, vergleichbar mit der Kamille.

Der bevorzugte Anteil dieser Wirkstoffe kann einzeln bis max. 10 Gew.-% bzw. bis 5 Gew.-% bzw. bis max. 2,5 Gew.-% betragen, wobei, sofern mehrere dieser Wirkstoffe enthalten sind, der Summenanteil an der Wirkstoffkombination max. 10 Gew.-% beträgt.

### Parfum:

Durch Parfumwirkstoffe wird der Wirkstoffkombination eine bestimmte Duftrichtung verliehen und kann damit auch der Grundgeruch der Wirkstoffkombination besser abgedeckt werden. Der bevorzugte Anteil an Parfuminhaltsstoffen beträgt bis max. 1,5 Gew.-% bzw. vorzugsweise zwischen 0,1 Gew.-% und 1 Gew.-%.

Um die durch Vermischen der einzelnen Inhaltsstoffe hergestellte Wirkstoffkombination besser verkapseln zu können kann, wie bereits oben ausgeführt wird, dieses Hautschutzöl noch verdünnt werden. Es sei hierzu auf obige Ausführungen verwiesen.

Des weiteren können von diesen Wirkstoffen auch Extrakte oder zumindest partiell hydratisierte Vertreter eingesetzt werden.

Die Wirkstoffkombination kann bis zu 98 Gew.-% zumindest eines der Hautpflegemittel enthalten. In diesem Falle sind Anteile an Konservierungsmittel, geruchshemmenden Mittel und Antioxidantien auf max. 2 Gew.-% in Summe beschränkt. Andererseits ist es möglich, dass die Wirkstoffkombination max. 90 Gew.-% an hautpflegenden Substanzen enthält, sodass also die anderen Inhaltsstoffe der Wirkstoffkombination einen Anteil von max. 10 Gew.-% haben.

Die Mengenanteile der Verdünnung beziehen auf die gesamte Wirkstoffkombiniation, d.h. dass die relativen Anteile der einzelnen Inhaltsstoffe in der Wirkstoffkombination zueinander von der Verdünnung nicht verändert werden.

Da es im Rahmen dieser Beschreibung nicht möglich ist, sämtliche Wirkstoffkombinationen wiederzugeben, werden im Folgenden lediglich die besonders bevorzugten Ausführungsbeispiele angeführt. Dabei sind die Angaben zu den Mengenanteilen in Gew.-% zu verstehen. In Klammer sind jeweils bevorzugte Bereiche der Zusammensetzungen angegeben, innerhalb derer die jeweiligen Inhaltsstoffe bzw. Wirkstoffe variiert werden können. Bei den Wirkstoffen handelt es sich um handelsübliche Produkte.

### Ausführungsbeispiel 1:

| | | |
|---|---|---|
| Caprylic/Capric Triglyceride | 96,0 % | (94 % - 98 %) |
| Benzylalkohol | 0,5 % | (0,1 % - 0,5 %) |
| Citronellol | 3,0 % | (0,1 % - 5 %) |
| Ascorbylpalmitat | 0,5 % | (0,05 % - 0,5 %) |

### Ausführungsbeispiel 2:

| | | |
|---|---|---|
| Extrakt der Sheabutter | 37 % | (20 % - 40 %) |
| Macadamiöl | 25 % | (10 % - 30 %) |
| Jojobaöl | 28,5 % | (10 % - 25 %) |
| Bisobolol oder Nikotinsäureamid | 2,5 % | (0,1 % - 3,0 %) |
| Ethylhexylstearat | 2,5 % | (1,5 % - 6,0 %) |
| Natriumhydroacetat | 0,5 % | (0,1 % - 0,5 %) |
| Rosmarinöl | 0,5 % | (0,1 % - 0,5 %) |

### Ausführungsbeispiel 3:

| | | |
|---|---|---|
| Avocadoöl | 25 % | (15,0 % - 25,0 %) |
| Macadamiöl | 31,7 % | (20,0 % - 35,0 %) |
| Jojobaöl | 25 % | (15,0 % - 25,0 %) |
| Parfum | 1 % | (0,1 % - 1 %) |
| Geraniol | 0,3 % | (- 0,3 %) |
| Haferextrakt | 5 % | (1,5 % - 8,0 %) |
| Ringelblumenöl | 5,7 % | (1,5 % - 8,0 %) |
| Natriumlaktat | 3 % | (1,0 % - 3,0 %) |
| Kaliumsorbat | 0,3 % | (0,01 - 0,3 %) |
| Ascorbylpalmitat | 0,5 % | (0,01 - 0,5 %) |
| Betacaroten | 0,25 % | (0,01 - 0,3 %) |
| Rosmarinöl | 0,25 % | (0,01 - 0,3 %) |

### Ausführungsbeispiel 4:

| | | |
|---|---|---|
| Caprylic/Capric Triglyceride | 96,0 % | (25 % - 50 %) |
| Persea Gratissima | 14,0 % | (10 % - 25 %) |
| Simmondsia Chinensis | 14,0 % | (10 % - 25 %) |
| Olea Europea Fruit Oil | 11,45 % | (10 % - 25 %) |
| Cytomethicone | 2,75 % | (1 % - 5 %) |
| Squalan | 2,75 % | (1 % - 5 %) |
| Borago Officinalis / Borage SE | 2,75 % | (1 % - 5 %) |
| Parfum | 0,5 % | (0,1 % - 1 %) |
| Bisabolol | 0,5 % | (0,1 % - 1 %) |
| Tocopheryl Acetat | 0,5 % | (0,1 % - 1%) |
| Linalool | 0,1 % | (- 0,1 %) |
| Limonene | 0,1 % | (-0,1%) |
| Buthylphenyl Methylpropional | 0,1% | (- 0,1 %) |
| Citronellol | 0,1 % | (-0,1%) |
| Coumarin | 0,1 % | (-0,1%) |
| Geraniol | 0,1 % | (- 0,1 %) |
| Citral | 0,1 % | (-0,1%) |
| Benzylalkohol | 0,5 % | (0,1 % - 0,5 %) |

### Ausführungsbeispiel 5:

| | |
|---|---|
| Borretschöl | 20,0 % |
| Squalan | 20,0 % |
| Avocadoöl | 25,0 % |
| Jojobaöl | 30 ,0 % |
| Kaliumsorbat | 0,5 % |
| Cumarin | 1,0 % |
| Lilial | 1,0 % |
| Tocopherylacetet | 0,5 % |
| Inositol | 2,0 % |
| Geraniol | 0,2 % |
| Arnikaextrakt | 0,1 % |
| Allantonin | 0,1 % |
| Parfum | 0,1 % |

### Ausführungsbeispiel 6:

| | |
|---|---|
| Macadamiaöl | 40,0 % |
| Olivenöl | 25,0 % |
| Squalan | 21,0 % |
| Kaliumsorbat | 0,5 % |
| Natriumlaktat | 3 % |
| Geraniol | 0,2 % |
| Kamillenextrakt | 10 % |
| Parfum | 0,3 % |

Mit diesen Wirkstoffkombinationen wurden, ggf. nach voranstehend beschriebener Verdünnung, Mikrokapseln hergestellt, mit einem dem Stand der Technik entsprechenden Verfahren zur Mikroverkapselung von Wirkstoffen. Diese Mikrokapseln wurden in der Folge einer üblichen, dem Stand der Technik entsprechenden Elastomercompoundierung in einer Konzentration zwischen 0,1 Gew.-% und 10 Gew.-%, bezogen auf die gesamte Latexdispersion, zugesetzt. Aus dieser wurden in der Folge Handschuhe mittels eines üblichen Tauchverfahrens, wie dies z.B. in den beiden oben genannten Dokumenten zum Stand der Technik der Anmelderin beschrieben ist, hergestellt.

Zur Beurteilung der Gebrauchseigenschaften wurden derartige Handschuhe mit Wirkstoffkombinationen entsprechend den Ausführungsbeispielen 1 bis 6 von jeweils 100 Testpersonen über einen Zeitdauer von jeweils 8 Stunden getragen. Jede dieser Testpersonen hat dabei jeweils einen Handschuh entsprechend den Ausführungsbeispielen 1 bis 6 getragen. Nach Ablauf der 8 Stunden wurde einerseits die Haut der Testpersonen visuell begutachtet, andererseits wurden mittels anonymisierter Fragenbögen, in denen Fragen hinsichtlich Juckreizgefühl während des Tragens, etc. gestellt wurden, subjektive Wahrnehmung dieser Testpersonen verifiziert. Es hat sich dabei herausgestellt, dass nahezu 100 % der Testpersonen einen Handschuh mit einer Wirkstoffkombination entsprechend Ausführungsbeispiel 4 am angenehmsten zu tragen empfunden haben. Ausführungen eines Handschuhs mit Wirkstoffkombinationen entsprechend den Ausführungsbeispielen 1 bis 3 sowie 5 und 6 haben zwischen 97 % und 99 % der Testpersonen mit sehr positiv hinsichtlich des Tragekomforts und des Gefühls auf der Haut beurteilt. Die visuelle Begutachtung der Haut nach dem Abziehen der Handschuhe von den Händen der Testpersonen zeigte in keinem Fall eine Rötung der Haut.

Die Wirkstoffkombinationen entsprechend den Ausführungsbeispielen 1 bis 6 haben alle eine ölartige Konsistenz.

Wie bereits voranstehend ausgeführt, ist zur Erhöhung der Lipidpenetration durch die Wirkstoffkombination, d.h. einzelne Bestandteile dieser Wirkstoffkombination, vorgesehen, dass die Viskosität dieser Wirkstoffkombination bei 37°C einen Wert von max. 250 mPas aufweist und eine Verseifungszahl nach DIN 53401 von max. 250. Zur Einstellung dieser Werte können die einzelnen Inhaltsstoffe entsprechend ihres Anteils an der Wirkstoffkombination variiert werden.

Zur weiteren Verbesserung der Lipidpenetration ist es von Vorteil, wenn der Anteil triglyceridischer Hautpflegemittel an der Wirkstoffkombination max. 50 Gew.-% beträgt.

Die Ausführungsbeispiele zeigen mögliche Ausführungsvarianten des Prophylaxeartikels, wobei an dieser Stelle bemerkt sei, dass auch diverse Kombinationen der einzelnen Ausführungsvarianten untereinander möglich sind.

## Patentansprüche

1. Prophylaxeartikel, insbesondere Handschuh, mit einem Trägerelement, das zumindest teilweise aus einem natürlichen oder synthetischen Elastomer besteht, sowie mit einer Wirkstoffkombination, die in Mikrokapseln enthalten ist, wobei die Mikrokapseln zumindest teilweise im und/oder auf dem Trägerelement angeordnet sind, **dadurch gekennzeichnet, dass** die Wirkstoffkombination zumindest ein Hautpflegemittel, zumindest ein Konservierungsmittel, zumindest ein geruchshemmendes Mittel und zumindest ein Antioxidans enthält, wobei das zumindest eine Hautpflegemittel ausgewählt ist aus einer Gruppe umfassend Triglyceride von Kokosfettsäuren, Avocadoöl, Jojobaöl, Olivenöl, Cyclomethicone, Squalan, Borretschöl, Sheabutter, Macadamiaöl, das zumindest eine Konservierungsmittel ausgewählt ist aus einer Gruppe umfassend Benzylalkohol, Benzylbenzoat, Kaliumsorbat, Natriumhydroacetat, das zumindest eine geruchshemmende Mittel ausgewählt ist aus einer Gruppe umfassend Citral, Cumarin, Citronellol, Lilial, Linalool, Limonene, das zumindest eine Antioxidans ausgewählt ist aus einer Gruppe umfassend Tocopheryl Acetat, Ascorbylpalmitat, Betacaroten, Rosmarinöl, wobei die Gruppen auch zumindest partiell hydratisierte Vertreter oder Extrakte der genannten Wirkstoffe umfassen und wobei die Wirkstoffkombination eine Verseifungszahl nach DIN 53401 von max. 250 aufweist.

2. Prophylaxeartikel nach Anspruch 1, **dadurch gekennzeichnet, dass** das oder die Konservierungsmittel, das oder die geruchshemmende(n) Mittel und das Antioxidans oder die Antioxidantien in Summe in einem Anteil von maximal 10 Gew.-% enthalten sind und den Rest das oder die Hautpflegemittel bilden.

3. Prophylaxeartikel nach Anspruch 1 oder 2, **dadurch gekennzeichnet, dass** die Wirkstoffkombination zumindest einen Feuchthaltefaktor enthält, der ausgewählt ist aus einer Gruppe umfassend Bisabolol, Fructose, Inositol, Nicotinsäureamid, Natriumlaktat.

4. Prophylaxeartikel nach Anspruch 3, **dadurch gekennzeichnet, dass** der Anteil des Feuchthaltefaktors oder der Feuchthaltefaktoren an der Wirkstoffkombination in Summe maximal 3 Gew.-% beträgt.

5. Prophylaxeartikel nach einem der Ansprüche 1 bis 4, **dadurch gekennzeichnet, dass** die Wirkstoffkombination Geraniol enthält.

6. Prophylaxeartikel nach Anspruch 5, **dadurch gekennzeichnet, dass** der Anteil an Geraniol in der Wirkstoffkombination maximal 0,3 Gew.-% beträgt.

7. Prophylaxeartikel nach einem der Ansprüche 1 bis 6, **dadurch gekennzeichnet, dass** die Wirkstoffkombination zumindest einen weiteren Wirkstoff ausgewählt aus einer Gruppe umfassend Allantonin, Arnikaextrakt, Haferextrakt, Ringelblumenöl, Kamillenextrakt, Ethylhexylstearat, Azulen enthält.

8. Prophylaxeartikel nach Anspruch 7, **dadurch gekennzeichnet, dass** der Anteil an dem oder an den weiterem(n) Wirkstoff(en) in Summe maximal 10 Gew.-% beträgt.

9. Prophylaxeartikel nach einem der Ansprüche 1 bis 8, **dadurch gekennzeichnet, dass** die Wirkstoffkombination zumindest ein Parfum enthält.

10. Prophylaxeartikel nach einem der Ansprüche 1 bis 9, **dadurch gekennzeichnet, dass** die Wirkstoffkombination mit einem Triglycerid und einem Duftstoff verdünnt ist, wobei ein Anteil des Triglycerids ausgewählt ist aus einem Bereich mit einer unteren Grenze von 10 Gew.-% und einer oberen Grenze von 20 Gew.-%, der Anteil des Duftstoffes ausgewählt ist aus einem Bereich mit einer unteren Grenze von 5 Gew.-% und einer oberen Grenze von 15 Gew.-%, und den Rest die Wirkstoffkombination bildet.

11. Prophylaxeartikel nach Anspruch 10, **dadurch gekennzeichnet, dass** der Duftstoff durch Lavendelöl gebildet ist.

12. Prophylaxeartikel nach einem der Ansprüche 1 bis 11, **dadurch gekennzeichnet, dass** das Trägerelement auf einer Oberfläche eine Gleitschicht aufweist.

13. Prophylaxeartikel nach Anspruch 12, **dadurch gekennzeichnet, dass** die Mikrokapseln zumindest teilweise in und/oder auf der Gleitschicht angeordnet sind.

14. Prophylaxeartikel nach einem der Ansprüche 1 bis 13, **dadurch gekennzeichnet, dass** die Wirkstoffkombination eine zumindest annähernd ölartige Konsistenz aufweist.

15. Prophylaxeartikel nach einem der Ansprüche 1 bis 14, **dadurch gekennzeichnet, dass** der Anteil triglyceridischer Hautpflegemittel an der Wirkstoffkombination maximal 50 Gew.- % beträgt.

## Claims

1. Prophylactic article, in particular a glove, with a carrier element, which comprises at least in part a natural or synthetic elastomer, and with a combination of active substances that is contained in microcapsules, and the microcapsules are arranged at least in part in and/or on the carrier element, **characterized in that** the combination of active substances contains at least one skin care agent, at least one preservative, at least one odor-inhibiting agent and at least one antioxidant, and the at least one skin care agent is selected from a group comprising triglycerides of coconut fatty acids, avocado oil, jojoba oil, olive oil, cyclomethicone, squalane, borage oil, shea butter, macadamia oil, the at least one preservative is selected from a group comprising benzyl alcohol, benzyl benzoate, potassium sorbate, sodium hydroacetate, the at least one odor-inhibiting agent is selected from a group comprising citral, cumarin, citronellol, lilial, linalool, limonene, the at least one antioxidant is selected from a group comprising tocopheryl acetate, ascorbyl palmitate, betacarotene, oil of rosemary, and the groups also comprise at least partially hydrated representatives or extracts of the cited active substances and the combination of active substances has a saponification value according to DIN 53401 of no more than 250.

2. Prophylactic article according to claim 1, **characterized in that** the preservative(s), the odor-inhibiting agent(s) and the antioxidant or the antioxidants in total are contained in a proportion of no more than 10% by weight and the skin care agent(s) form the rest.

3. Prophylactic article according to claim 1 or 2, **characterized in that** the combination of active substances contains at least one humectant factor, which is selected from a group comprising bisabolol, fructose, inositol, nicotinamide, sodium lactate.

4. Prophylactic article according to claim 3, **characterized in that** the proportion of the humectant factor or the humectant factors in the combination of active substances in total is no more than 3% by weight.

5. Prophylactic article according to one of claims 1 to 4, **characterized in that** the combination of active substances contains geraniol.

6. Prophylactic article according to claim 5, **characterized in that** the proportion of geraniol in the combination of active substances is a max. of 0.3% by weight.

7. Prophylactic article according to one of claims 1 to 6, **characterized in that** the combination of active substances contains at least one further active substance selected from a group comprising allantonin, arnica extract, oat extract, calendula oil, chamomile extract, ethylhexyl stearate, azulene.

8. Prophylactic article according to claim 7, **characterized in that** the proportion further active substance(s) in total is no more than 10% by weight.

9. Prophylactic article according to one of claims 1 to 8, **characterized in that** the combination of active substances contains at least one perfume.

10. Prophylactic article according to one of claims 1 to 9, **characterized in that** the combination of active substances is diluted with a triglyceride and a perfume, and a proportion of the triglyceride is selected from a range with a lower limit of 10% by weight and an upper limit of 20% by weight, the proportion of perfume is selected from a range with a lower limit of 5% by weight and an upper limit of 15% by weight, and the combination of active substances forms the rest.

11. Prophylactic article according to claim 19, **characterized in that** the perfume is provided in the form of lavender oil.

12. Prophylactic article according to one of claims 1 to 11, **characterized in that** the carrier element has a sliding layer on a surface.

13. Prophylactic article according to claim 12, **characterized in that** the microcapsules are arranged at least in part in and/or on the sliding layer.

14. Prophylactic article according to one of claims 1 to 13, **characterized in that** the combination of active substances has an at least approximately oily consistency.

15. Prophylactic article according to one of claims 1 to 14, **characterized in that** the proportion of triglyceride skin care agents in the combination of active substances is no more than 50% by weight.

## Revendications

1. Article prophylactique, notamment gant, avec un élément support qui est constitué au moins en partie en un élastomère naturel ou synthétique, ainsi qu'avec une combinaison de substances actives qui est enfermée dans des microcapsules, les microcapsules étant au moins en partie disposées dans et/ou sur l'élément support, **caractérisé en ce que** la combinaison de substances actives comprend au moins un produit de soin de la peau, au moins un agent conservateur, au moins un agent désodorant et au moins un antioxydant, le ou les produits de soin de la peau étant choisis dans un groupe comportant du triglycéride d'acides gras de coco, de l'huile d'avocat, de l'huile de jojoba, de l'huile d'olives, de la cyclométhicone, du squalane, de l'huile de bourrache, du beurre de karaté, de l'huile de macadamia, le ou les agents conservateurs étant choisis dans un groupe comportant de l'alcool benzylique, du benzoate de benzyle, du sorbate de potassium, de l'acétate hydrique de sodium, le ou les agents désodorants étant choisis dans un groupe comportant du citral, de la coumarine, du citronellol, du lilial, du linalool, de la limonene, le ou les antioxydants étant choisis dans un groupe comportant l'acétate de tocophéryle, du palmitate d'ascorbyle, de la bêta-carotène, de l'huile de romarin, les groupes comprenant aussi des représentants ou extraits au moins partiellement hydratés des substances actives désignées et la combinaison des substances actives ayant un indice de saponification de 250 au maximum selon la norme DIN 53401.

2. Article prophylactique selon la revendication 1, **caractérisé en ce que** le ou les agents conservateurs, le ou les agents désodorants et le ou les antioxydants sont contenus en somme dans une partie de 10 % en poids au maximum et que le reste est constitué par le ou les produits de soin de la peau.

3. Article prophylactique selon la revendication 1 ou 2, **caractérisé en ce que** la combinaison de substances actives comprend au moins un agent hydratant qui est choisi dans un groupe comportant du bisabolol, du fructose, de l'inositol, de l'amide d'acide nicotinique, du lactate de sodium.

4. Article prophylactique selon la revendication 3, **caractérisé en ce que** le pourcentage de l'agent hydratant ou des agents hydratants dans la combinaison de substances actives est en somme au maximum de 3 % en poids.

5. Article prophylactique selon l'une des revendications 1 à 4, **caractérisé en ce que** la combinaison de substance actives comprend du géraniol.

6. Article prophylactique selon la revendication 5, **caractérisé en ce que** le pourcentage du géraniol dans la combinaison de substances actives est en somme au maximum de 0,3 % en poids.

7. Article prophylactique selon l'une des revendications 1 à 6, **caractérisé en ce que** la combinaison de substances comprend au moins une autre substance active choisie dans un groupe comportant de l'allantoïne, de l'extrait d'arnica, de l'extrait d'avoine, de l'huile de calendula, de l'extrait de camomille, du stéarate d'éthylhexyle, de l'azulène.

8. Article prophylactique selon la revendication 7, **caractérisé en ce que** le pourcentage de la ou des substances actives supplémentaires est en somme au maximum de 10 % en poids.

9. Article prophylactique selon l'une des revendications 1 à 8, **caractérisé en ce que** la combinaison de substances actives comprend au moins un parfum.

10. Article prophylactique selon l'une des revendications 1 à 9, **caractérisé en ce que** la combinaison de substances actives est diluée avec un triglycéride et un agent de fragrance, une teneur en triglycéride étant choisie dans une plage ayant une limite inférieure de 10 % en poids et une limite supérieure de 20 % en poids, la teneur en agent de fragrance étant choisie dans une plage ayant une limite inférieure de 5 % en poids et une limite supérieure de 15 % en poids et le reste étant constitué par la combinaison de substances actives.

11. Article prophylactique selon la revendication 10, **caractérisé en ce que** l'agent de fragrance est constitué par de l'huile de lavande.

12. Article prophylactique selon l'une des revendications 1 à 11, **caractérisé en ce que** l'élément support comprend sur une surface une couche de glissement.

13. Article prophylactique selon la revendication 12, **caractérisé en ce que** les microcapsules sont disposées au moins partiellement dans et/ou sur la couche de glissement.

14. Article prophylactique selon l'une des revendications 1 à 13, **caractérisé en ce que** la combinaison de substances présente une consistance au moins approximativement huileuse.

15. Article prophylactique selon l'une des revendications 1 à 14, **caractérisé en ce que** le pourcentage de produits triglycéridiques de soin de la peau dans la combinaison de substances actives est au maximum de 50 % en poids.
